(19) **Europäisches Patentamt**

**European Patent Office**

**Office européen des brevets**

(11) **EP 1 096 004 B1**

(12) **EUROPEAN PATENT SPECIFICATION**

(45) Date of publication and mention
of the grant of the patent:
**15.09.2004 Bulletin 2004/38**

(51) Int Cl.⁷: $C11D\ 3/50$, $C07C\ 43/303$, $C07C\ 43/305$, $C07C\ 43/307$, $A61K\ 7/46$

(21) Application number: **00204671.2**

(22) Date of filing: **22.03.1996**

(54) **Pro-fragrance compounds**

Duftstoffvorläufer

Précurseurs de parfum

(84) Designated Contracting States:
**AT BE CH DE DK ES FI FR GB GR IE IT LI LU NL PT SE**

(43) Date of publication of application:
**02.05.2001 Bulletin 2001/18**

(62) Document number(s) of the earlier application(s) in accordance with Art. 76 EPC:
**96910554.3 / 0 888 439**

(73) Proprietor: **THE PROCTER & GAMBLE COMPANY Cincinnati, Ohio 45202 (US)**

(72) Inventors:
• **Mao, Hsiang Kuen
Kobe 658 (JP)**
• **Na, Henry Cheng
Wyoming, Ohio 45215 (US)**
• **Sivik, Mark Robert
Mason, Ohio 45040, Warren County (US)**

• **Morelli, Joseph Paul
Cincinnati, Ohio 45220 (US)**
• **Pan, Robert Ya-Lin
Cincinnati, Ohio 45242 (US)**

(74) Representative: **Mather, Peter Geoffrey et al
NV Procter & Gamble Services Company SA,
100 Temselaan
1853 Strombeek-Bever (BE)**

(56) References cited:
DE-A- 2 132 898          US-A- 5 378 468
US-A- 5 500 154

• **MAHADEVAN V: "Reactions of fatty aldehydes with fatty alcohols" LIPIDS, vol. 5, no. 6, 1970, pages 563-565, XP000987285**

Remarks:
The file contains technical information submitted after the application was filed and not included in this specification

**Description**

FIELD OF THE INVENTION

The present invention relates to a pro-fragrance compound. More particularly, the invention relates to a pro-fragrance, which may be used in detergent compositions. Those detergent compositions may be used for accomplishing the delivery of such organic pro-fragrance compound to textile articles and other surfaces washed with said compositions. The delivery of the fragrances is a delayed release, when the surfaces are washed in an aqueous bath in the presence of conventional detergent ingredients. The fragrance is released in fragrance-active form when the surface is in contact with a lower pH environment such as contact with water, carbon dioxide gas, humid air, or the like.

BACKGROUND OF THE INVENTION

Most consumers have come to expect scented laundry products and to expect that fabrics, which have been laundered to also have a pleasing fragrance. It is also desired by consumers for laundered fabrics to maintain the pleasing fragrance over time. Perfume additives make laundry compositions more aesthetically pleasing to the consumer, and in some cases the perfume imparts a pleasant fragrance to fabrics treated therewith. However, the amount of perfume carry-over from an aqueous laundry bath onto fabrics is often marginal and does not last long on the fabric. In addition, some perfume delivery systems are not stable under alkaline conditions, such as in laundry detergent compositions. Fragrance materials are often very costly and their inefficient use in detergents and ineffective delivery to fabrics from detergents results in a very high cost to both consumers and detergent manufacturers. Industry, therefore, continues to seek with urgency for more efficient and effective fragrance delivery in laundry products, especially for improvement in the provision of long-lasting fragrance to the laundered fabrics.

US-A-5 500 154 relates to detergent compositions comprising an enduring perfume composition, the ingredients of which having boiling points of 250°C or higher, and a Clog P of about 3 or higher.

Acetals and ketals have long been known in perfumery. See Steffen Arctander, "Perfume and Flavor Chemicals", Arctander, N.J., 1969. The majority of these are methyl and ethyl types, and molecular weights may range widely. See, for example, Arctander abstract numbers 6, 11, 210, 651, 689, 1697, 1702, 2480, 2478. For 2478, which is phenylacetaldehyde dicitronellyl acetal, molecular weight 414.7, Arctander reports " ... and it is not exaggerated to say that this acetal is practically abandoned and obsolete in today's perfumery". For 2480, which is phenylacetaldehyde digeranyl acetal, Arctander reports " the title material does not offer substantial advantages or unique odor type and it may be considered of little more than academic interest today". This latter material was still commercially available in 1992 as ROSETAL A (Catalogue, IFF). The present inventors have found indeed that the acetals of aldehydes, which have low molecular weight and contain a $C_6H_5$ moiety, such as benzaldehyde and phenylacetaldehyde, do not have very desirable odor character for use in a pro-fragrancing detergent mode. Yet another group of commercial acetals sold for incorporation in perfumes are those of undecylenic aldehyde, such as the digeranyl or dicitronellyl acetals. The present inventors have found that these materials too are not very desirable for use in profragrancing detergent compositions.

U.S. Patent 2,490,337, Croxall et al, issued Dec. 6, 1949 describes a method for preparing ketals. A synthesis is presented, in which isopropenyl acetate is reacted with at least two equivalents of an alcohol in the presence of a mercury compound and a strongly acidic catalyst to give the corresponding ketal. In fact, these ketals always contain a propyl-$C_3$ carbon chain. The range of different alcohols, which may be used in this method, is also restricted to those, which are primary, or secondary, and to those, which do not contain any interfering group (amino group), which reacts with or destroys the acid catalyst. However, as additional subclass functional groups such as ether or ester groups bound to the carbon chain of the alcohol are tolerated.

U.S. Patent 2,448,660, Croxall et al, issued Sept. 7, 1948 describes a method for preparing acetals. A synthesis is presented, in which a vinyl ester of a carboxylic acid is reacted with at least two equivalents of an alcohol in the presence of a mercury compound and a strongly acidic catalyst to give the corresponding acetal. As a matter of fact, these acetals always contain an ethyl-$C_2$ carbon chain. The range of different alcohols, which may be used in this method, is also restricted to those, which are primary, or secondary, and to those, which do not contain any interfering group (amino group), which reacts with or destroys the acid catalyst. The alcohols used in this preparation may be aliphatic, cycloaliphatic, arylaliphatic, or heterocyclic aliphatic. In all of these compounds, the OH-group has to be attached to a -$CH_2$- or to a -CH= group, that means, it is aliphatically bound and not directly attached to a carbon atom of an aryl group. The claims of this U.S. Patent are only related to ether acetals containing between one to twelve alkoxy groups of two to three carbon atoms each; the patent is not related to acetals or ketals themselves. Consequently, each granted compound following this U.S. Patent must contain at least one ethoxy or (iso)propoxy group. This patent is also related to polyhydroxy alcohols. However, compounds with only two -OH groups have been disclosed.

U.S. Patent 2,668,862, Price et al, issued Feb. 9, 1954 describes a method for preparing acetals. A synthesis is presented, in which an aldehyde is reacted with at least two equivalents of an alcohol in the presence of a strongly acidic catalyst and of an anhydrous salt for removal of the water of reaction to give the corresponding acetal. The acetal has to consist of twelve to 42 carbon atoms. The preparation of $C_{24}$ to $C_{36}$ branched chain acetals from oxo products, obtained by oxogenation of an olefin with carbon monoxide to an aldehyde, which is then hydrogenated to the corresponding alcohol as parent alcohol for the cited patent, is described.

None of these patents mention that at least one of the parent compounds, e.g., the parent alcohol, aldehyde and/or ketone and therefore the resulting acetal or ketal has to be a fragrance or a pro-fragrance. Consequently, the use of the prepared compounds as pro-fragrance for a personal care or for a laundry product in a washing process is not suggested. In fact, all cited patents silent to the demanding task to fix a fragrance onto a surface, especially to fabrics.

U.S. Patent 5,378,468, Suffis et al, issued Jan. 3, 1995 describes specific types of personal care compositions, such as deodorant sticks, comprising assertedly "body-activated" fragrances. The term apparently refers to the previously known tendency of materials such as acetals derived from fragrance alcohols to hydrolyze under acidic pH conditions thereby releasing fragrance. See, for example, U.S. 3,932,520, Hoffman, issued January 13, 1976.

Factors affecting substantivity of fragrance materials on fabrics are discussed in Estcher et al. JAOCS 71 p. 31-40 (1994).

The selected potential fragrance materials described by Suffis et al include particular acetals and ketals, exemplified by propylene glycol vanillin acetal. The materials exemplified apparently are rather hydrophilic short chain alcohol or diol derivatives of fragrance aldehydes and upon hydrolysis, deliver one mole of the aldehyde per mole of the potential fragrance material. The present inventors believe that hydrophilic acetal or ketal materials, i.e., those having a CLogP value (described hereafter) of less than 4 have at best limited usefulness in laundry detergent compositions. The Suffis et al development is designed to be incorporated with a personal care product vehicle, resulting in clear deodorant sticks and the like.

For detergent use, it is important that rather hydrophobic pro-fragrant compounds be used in order to enhance deposition onto surfaces in the wash solution and retention on the washed surface during rinsing. In Suffis et al, the compositions containing the potential fragrance materials are applied directly to the substrate (i.e. skin); therefore, the deposition problems resulting from dilution, rinsing, etc. are not at issue.

More specifically, in contrast to deodorant sticks and the like, laundry detergents are used in dilute aqueous form and contain numerous detergent adjuncts such as synthetic detergents, builders, enzymes and the like which are capable of micellizing, or solubilizing the pro-fragrance. Further, in order to remove detergent adjuncts and the soils displaced by detergent adjuncts from the fabrics, the latter are rinsed after washing. The rinsing tends to remove the useful pro-fragrance material deposited. Thus both the detergent adjuncts and the essential steps of the wash process itself all work against the effective delivery of pro-fragrances to the fabrics being washed. Moreover, high-efficiency pro-fragrant systems are desired for laundry purposes. In many laundry applications, the use of heated tumble-drying appliances further exacerbates the problem of delivering adequate residual fragrance to textile fabric surfaces. Suffis et al are silent on both the nature of these severe technical problems and shortcomings, as well as methods and specific pro-fragrances to overcome them.

It has now surprisingly been discovered that these problems can unexpectedly be overcome by the selection of specific organic pro-fragrance types. Moreover, when these pro-fragrance types are selected, a simple but effective method is successfully provided for their effective delivery. Accordingly, objects of the present invention include the provision of such pro-fragrance types.

By the term "pro-fragrance" herein, it is meant a compound which may or may not be odoriferous in itself but which, upon hydrolysis, produces a desirable odor which is characteristic of one or more of its hydrolysis products. Of course, mixtures of pro-fragrance compounds can also be considered a pro-fragrance.

## SUMMARY OF THE INVENTION

The present invention relates to a pro-fragrance compound containing acetal, which may be used in detergent compositions. Those detergent compositions may impart residual fragrances to surfaces washed with aqueous solutions of said pro-fragrance. The delivery of the fragrances is a delayed release, when the surfaces are washed in an aqueous bath in the presence of conventional detergent ingredients.

The present invention relates to a pro-fragrance compound selected from the group consisting of acetals, ketals, and mixtures thereof, wherein at least one of the parent aldehydes, ketones, or alcohols of said pro-fragrant acetal or ketal is a fragrance compound, said pro-fragrant compound having;

(i) a molecular weight of at least about 350,
(ii) a CLogP of at least about 4, wherein CLogP is the logarithm to base 10 of the Octanol/Water Partition Coefficient of said pro-fragrant compound, and
(iii) a half-life of less than 60 minutes, when measured at pH 0 by the Pro-Fragrant Hydrolysis Test;

provided that said parent aldehyde, ketone or alcohol of said acetal or ketal comprises at least one compound selected from the group consisting of;

a) aldehydes, ketones and alcohols containing at least one aromatic moiety selected from the group consisting of $C_6H_4$ and $C_6H_3$ and wherein said parent aldehyde or ketone has a molecular weight of at least 125;
b) monoalcohols selected from the group consisting of $C_{11}$-$C_{20}$ saturated, unsaturated, aromatic, aliphatic linear and branch chain alcohols and alkoxylates of said alcohols containing from 1 to about 30 alkoxy groups wherein said alkoxy is selected from the group consisting of ethoxy, propoxy and butoxy and mixtures thereof;
c) polyhydroxy alcohols, and
d) mixtures thereof;

wherein said pro-fragrance compound is digeranyl p-t-bucinal acetal.

DETAILED DESCRIPTION OF THE INVENTION

Pro-fragrances

The pro-fragrance of this invention is an acetal namely digeranyl p-t-bucinal acetal. Acetals may in general be considered as derivable from aldehydes in combination with alcohols. These aldehydes and alcohols are herein termed "parents" or "parent compounds" of the acetal. At least one parent of any of the instant acetals is a fragrance compound. Additionally the pro-fragrance compound of the inventive compositions has the following properties:

(i) molecular weight of at least 350,
(ii) CLogP of at least 4, (preferably at least 6, more preferably at least 10) wherein CLogP is the logarithm to base 10 of the octanol/water partition coefficient of said pro-fragrant compound, and
(iii) a half-life of less than 60 minutes, when measured at pH 0 by the Pro-Fragrant Hydrolysis Test.

The pro-fragrance compound is stable under pH conditions encountered in the formulation and storage of detergent products, which have a pH of from 7.1 to 13, and during solution-use of such products. Due to its high molecular weight and hydrophobicity, the pro-fragrance compound gives reasonably good deposition from a laundering solution onto fabrics. Because the pro-fragrant compound is subject to hydrolysis when the pH is reduced, it hydrolyzes to release its component fragrance compounds when the fabrics upon which it has been deposited are exposed even to reduced pH such as present in rinse water, air and humidity. The reduction in pH should be at least 0.1, preferably at least 0.5 units. Preferably the pH is reduced by at least 0.5 units to a pH of 7.5 or less, more preferably 6.9 or less. Preferably, the solution in which the fabric (or other surface) is washed is alkaline.
The acetal herein is derived from a parent aldehyde other than those, which possess all of the following characteristics:

(a) low molecular weight;
(b) contain a $C_6H_5$ moiety which has no substituent groups other than the aldehyde itself (Such relatively undesirable acetals for the present purposes are those derived from benzaldehyde and phenylacetaldehyde. More preferably, acetals herein, when they comprise an aromatic moiety, will be derived from a parent aldehyde having molecular weight above 125, more preferably above 140.);
(c) contain a $C_1$ or a $C_2$ carbon chain.

The essential pro-fragrance component herein can be used at widely ranging levels. The pro-fragrant acetal is formulated in detergent compositions at levels in the general range 0.0001% to 10%, more preferably from 0.001% to 5%, more preferably still, from 0.01% to 1%.
A pro-fragrance can be used as the sole fragrance component of detergent compositions, or in combination with other pro-fragrances and/or in combination with other fragrance materials, extenders, fixatives, diluents and the like. For example, incorporation of the pro-fragrant material into a waxy substance, such as a fatty triglyceride may further improve storage stability of the present pro-fragrant compound in granular laundry detergents, especially those comprising bleach. In liquid or gel forms of detergent compositions, hydrophobic liquid extenders, diluents or fixatives can be used to form an emulsion wherein the pro-fragrant compound is further stabilized by separating it from the aqueous phase. Nonlimiting examples of such stabilizing materials include dipropylene glycol, diethyl phthalate and acetyl triethyl citrate. Just as there exist hydrophobic perfumery ingredients which can be used to stabilize the pro-fragrant material, there also exist detergency ingredients which also have a perfume stabilizing effect and can be formulated with the pro-fragrant material. Such ingredients include fatty acid amines, low foaming waxy nonionic materials commonly used in automatic dishwashing detergents, and the like. In general where pro-fragrances are used along with other fragrance materials in detergent compositions herein it is preferred that the pro-fragrance be added separately from the other fragrance materials.

Synthesis of pro-fragrances

Acetals can be prepared by the acid catalyzed reaction of an aldehyde with an alcohol (or diol), using conventional acid catalysis such as HCl or p-toluenesulfonic acid, or supported sulfonic acid catalysts e.g., AMBERLYST 15™. See Meskens, F., *Synthesis,* (7) 501 (1981) and Meskens, F., *Jannsen Chim Acta* (1) 10 (1983). Many aldehyde and alcohols useful in the synthesis of acetal pro-fragrance of the present invention are sensitive to strong acid conditions and can undergo undesirable side reactions. See Bunton, C.A. et al, *J. Org. Chem.* (44), 3238, (1978), and Cort, O., et al, *J. Org. Chem.* (51), 1310 (1986). It is also known that acetals of *alpha, beta* unsaturated aldehydes can undergo migration of the double bond under the inappropriate selection of the acid catalyst. See Meskens, F., *Synthesis,* (7), 501, (1981) and Lu, T.-J, et al. *J. Org. Chem.* (60), 2931, (1995). For acid sensitive materials, acid catalysts with pKa's between 3 and 4 are the most desirable to minimize double bond migration while maintaining the reactivity necessary to produce the acetal.

Another technique of avoiding side reactions in preparing acetals of acid sensitive materials, such as geraniol, is by transacetalization of a dimethyl acetal with a higher molecular weight alcohol, using a mild Lewis acid such as titanium isopropoxide or boron trifluoride etherate as the catalyst.

Test Methods

Calculation of CLogP

The pro-fragrance of the invention is characterized by its octanol/water partition coefficient P. The octanol/water partition coefficient of a pro-fragrance is the ratio between its equilibrium concentration in octanol and in water. Since the partition coefficients of the pro-fragrance compound is large, it is more conveniently given in the form of its logarithm to the base 10, logP.

The logP of many compounds have been reported; for example, the Pomona92 database, available from Daylight Chemical Information Systems, Inc. (Daylight CIS), contains many, along with citations to the original literature.

However, the logP values are most conveniently calculated by the "CLOGP" program, also available from Daylight CIS. This program also lists experimental logP values when they are available in the Pomona92 database. The "calculated logP" (CLogP) is determined by the fragment approach of Hansch and Leo (cf., A. Leo, in Comprehensive Medicinal Chemistry, Vol. 4, C. Hansch, P.G. Sammens, J.B. Taylor and C.A. Ramsden, Eds., p. 295, Pergamon Press, 1990). The fragment approach is based on the chemical structure of a compound and takes into account the numbers and type of atoms, the atom connectivity, and chemical bonding. The CLogP values, which are the most reliable and widely used estimates for this physicochemical property, can be used instead of the experimental logP values in the selection of pro-fragrances.

Determination of Hydrolysis Half-life (t-1/2)

Hydrolysis half-life is the measurement used to determine the ease with which the pro-fragrance compound undergoes acid hydrolysis and thereby releases its fragrance component(s) upon exposure to acid conditions. The pro-fragrant compound of the invention has a half-life of less than 60 minutes, under the described hydrolysis conditions at pH 0. Preferably, pro-fragrance of the invention has a half-life at pH 2 of less than 60 minutes. For granular detergents, the more reactive pro-fragrances, that is, those with half-life at pH 2 of less than one minute, are most suitable, although those having a half-life of less than 60 minutes at pH 0 are also useful. For liquid detergent applications, pro-fragrances having a half-life of less than 60 minutes at pH 0, and half-life greater than one minute at pH 2 should preferably be used.

Hydrolysis half-life is determined by UV/Vis spectroscopy in a 90/10 dioxane/water system at 30°C by following the appearance of the carbonyl absorbance. Because of the hydrophobicity of the pro-fragrance compounds of the invention, a high dioxane/water ratio is needed to ensure solubility of the pro-fragrance. The pH of the water used is achieved by using aqueous HCl. The concentration of the pro-fragrance in the dioxane/water system can be adjusted to achieve convenient, measurable absorbance changes.

All measurements are carried out using a Hewlett Packard 8452 A Diode Array Spectrophotometer using quartz 1 cm path length cuvette cells. Materials used include 1,4-dioxane HPLC Grade 99.9% (Sigma-Aldrich), 1N HCl volurnetric solution (J.T. Baker), deionized water filtered with MilliQPlus (Millipore) at resistivity of 18.2 M Ohm cm. The pH's are measured using an Orion 230 A standardized with pH 4 and pH 7 buffers. The 1N HCl standard is used directly for pH 0 conditions. For pH 2 conditions, 1N HCl is diluted with deionized water.

Pro-fragrance is weighed out in a 10.00 ml volumetric flask using an analytical balance (Mettler AE 200) Precision is 1/10 mg. The weighed material is dissolved in 8 ml dioxane. Both the dioxane solution of pro-fragrance and aqueous acid solution prepared as described supra are pre-heated in their separate containers to a temperature of 30 ± 0.25 °C by means of a water-bath. 1.000 ml of aqueous acid solution is added to the pro-fragrance solution by means of an Eppendorf pipetter. This is followed by diluting to the 10.00 ml mark with dioxane. Hydrolysis time is measured, starting upon addition of the acid. The pro-fragrance solution is mixed for 30 seconds by shaking, and the solution is transferred to a quartz cuvette. The absorbance of the pro-fragrance solution ($A_t$) is followed at a regular series of time intervals, and the cuvette is kept in the water-bath at the above-indicated temperature between measurements. Initial absorbance ($A_o$) measurements are carried out using an equal concentration of pro-fragrance in a 90/10 v/v dioxane - deionized water solution, and final absorbance ($A_f$) measurements are taken using the hydrolyzed pro-fragrance solution after the hydrolysis is complete. The wavelength at which the hydrolysis is followed is chosen at the wavelength of the absorbance maximum of the parent aldehyde or ketone.

Reaction half-lifes are determined using conventional procedures. The observed first-order rate constant ($k_{obs}$) is determined by slope of the line provided by plotting the following function vs time (min):

$$Ln \left[ (A_o - A_f) / (A_t - A_f) \right]$$

wherein said function is the natural log of the ratio between the absorbance difference at initial time ($A_o$) and final time ($A_f$) over the absorbance difference at time t ($A_t$) and final time ($A_f$).

Half-life as defined herein is the time required for half of the pro-fragrance to be hydrolyzed, and is determined from the observed rate constant ($k_{obs}$) by the following function:

$$Ln \, (1/2) = -k_{obs} \, t_{1/2}$$

Conventional Detergent Ingredients

The following are illustrative examples of detersive surfactants and other detergent ingredients.

Detersive Surfactants

Non-limiting examples of synthetic detersive surfactants useful herein typically at levels from 0.5% to 90%, by weight, include the conventional $C_{11}$-$C_{18}$ alkyl benzene sulfonates ("LAS") and primary, branched-chain and random $C_{10}$-$C_{20}$ alkyl sulfates ("AS"), the $C_{10}$-$C_{18}$ secondary (2,3) alkyl sulfates of the formula $CH_3(CH_2)_x(CH(CH_3)OSO_3^-M^+)$ and $CH_3(CH_2)_y(CH(CH_2CH_3)OSO_3^-M^+)$ wherein x and y are integers and wherein each of x and (y + 1) is least 7, preferably at least 9, and M is a water-solubilizing cation, especially sodium, unsaturated sulfates such as oleyl sulfate, the $C_{10}$-$C_{18}$ alkyl alkoxy sulfates ("$AE_XS$"; especially EO 1-7 ethoxy sulfates), $C_{10}$-$C_{18}$ alkyl alkoxy carboxylates (especially the EO 1-5 ethoxycarboxylates), the $C_{10}$-$C_{18}$ glycerol ethers, the $C_{10}$-$C_{18}$ alkyl polyglycosides and their corresponding sulfated polyglycosides, and $C_{12}$-$C_{18}$ alpha-sulfonated fatty acid esters. If desired, the conventional nonionic and amphoteric surfactants such as the $C_{12}$-$C_{18}$ alkyl ethoxylates ("AE") including the so-called narrow peaked alkyl ethoxylates and $C_6$-$C_{12}$ alkyl phenol alkoxylates (especially ethoxylates and mixed ethoxylpropoxylates), $C_{12}$-$C_{18}$ betaines and sulfobetaines ("sultaines"), $C_{10}$-$C_{18}$ amine oxides, and the like, can also be included in the overall compositions. The $C_{10}$-$C_{18}$ N-alkyl polyhydroxy fatty acid amides can also be used. Typical examples include the $C_{12}$-$C_{18}$ N-methylglucamides. See WO 9,206,154. Other sugar-derived surfactant include the N-alkoxy polyhydroxy fatty acid amides, such as $C_{10}$-$C_{18}$ N-(3-methoxypropyl) glucamide. The N-propyl through N-hexyl $C_{12}$-$C_{18}$ glucamides can be used for low sudsing. $C_{10}$-$C_{20}$ conventional soaps may also be used, however synthetic detergents are preferred. If high sudsing is desired, the branched-chain $C_{10}$-$C_{16}$ soaps may be used. Mixtures of anionic and nonionic surfactants are especially useful. Other conventional useful surfactants are listed in standard texts. See also U.S. Patent 3,664,961, Norris, issued May 23, 1972.

Preferred compositions incorporating only synthetic detergents have a detergent level of from 0.5% to 50%. Compositions containing soap preferably comprise from 10% to 90% soap.

Although the detergent compositions herein can consist of only detersive surfactant and pro-fragrance, the said compositions preferably contain other ingredients commonly used in detergent products.

Builders

Detergent builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end use of the composition and its desired physical form. When present, the compositions will typically comprise at least 1% builder. Liquid formulations typically comprise from 5% to 50%, more typically 5% to 30%, by weight, of detergent builder. Granular formulations typically comprise from 10% to 80%, more typically from 15% to 50% by weight, of the detergent builder. Lower or higher levels of builder, however, are not meant to be excluded.

Inorganic or detergent builders include, but are not limited to phosphate builders such as, the alkali metal, ammonium and alkanolammonium salts of polyphosphates (exemplified by the tripolyphosphates, pyrophosphates, and glassy polymeric meta-phosphates), phosphonates, and phytic acid, and non-phosphorous builders such as silicates, carbonates (including bicarbonates and sesquicarbonates), sulphates, and aluminosilicates. Non-phosphate builders are required in some locales.

Organic builders suitable for use herein include polycarboxylate builders such as disclosed in U.S. Patent 3,308,067, Diehl issued March 7, 1967; 4,144,226, Crutchfield issued March 13, 1979 and 4,246,495, Crutchfield, issued March 27, 1979.

Soil Release Agents

Soil Release agents are desirably used in laundry detergents. Suitable soil release agents include those of U.S. 4,968,451, November 6, 1990 to J.J. Scheibel and E.P. Gosselink: such ester oligomers can be prepared by (a) ethoxylating allyl alcohol, (b) reacting the product of (a) with dimethyl terephthalate ("DMT") and 1,2-propylene glycol ("PG") in a two-stage transesterification/oligomerization procedure and (c) reacting the product of (b) with sodium metabisulfite in water; the nonionic end-capped 1,2-propylene/polyoxyethylene terephthalate polyesters of U.S. 4,711,730, December 8, 1987 to Gosselink et al, for example those produced by transesterification/oligomerization of poly(ethyleneglycol) methyl ether, DMT, PG and poly(ethyleneglycol) ("PEG"); the partly- and fully- anionic-end-capped oligomeric esters of U.S. 4,721,580, January 26, 1988 to Gosselink, such as oligomers from ethylene glycol ("EG"), PG, DMT and Na-3,6-dioxa-8-hydroxyoctanesulfonate; the nonionic-capped block polyester oligomeric compounds of U.S. 4,702,857, October 27, 1987 to Gosselink, for example produced from DMT, Me-capped PEG and EG and/or PG, or a combination of DMT, EG and/or PG, Me-capped PEG and Na-dimethyl-5-sulfoisophthalate; and the anionic, especially sulfoaroyl, end-capped terephthalate esters of U.S. 4,877,896, October 31, 1989 to Maldonado, Gosselink et al, the latter being typical of SRA's useful in both laundry and fabric conditioning products, an example being an ester composition made from m-sulfobenzoic acid monosodium salt, PG and DMT optionally but preferably further comprising added PEG, e.g., PEG 3400. Another preferred soil release agent is a sulfonated end-capped type described in US 5,415,807.

Other Optional Ingredients

The compositions herein can contain other ingredients such as enzymes, bleaches, fabric softening agents, dye transfer inhibitors, suds suppressors, and chelating agents, all well known within the art.

For purposes of defining detergent compositions, the pH of the detergent composition is that which is measured at 1% concentration of the detergent composition in distilled-water at 20°C. The detergent compositions herein have a pH of from 7.1 to 13, more typically from 7.5 to 9.5 for liquid detergents and from 8 to 12 for granular detergents.

Formulation with Detergents With or Without Conventional Perfumery Materials

While the pro-fragrance of the present invention can be used alone and simply mixed with essential detergent ingredient, most notably surfactant, it can also be desirably combined into three-part formulations which combine (a) a non-fragranced detergent base comprising one or more synthetic detergents, (b) the pro-fragrant acetal in accordance with the invention and (c) a fully-formulated fragrance. The latter provides desirable in-package and in-use (wash-time) fragrance, while the pro-fragrance provides a long-term fragrance to the laundered textile fabrics.

In formulating the present detergents, the fully-formulated fragrance can be prepared using numerous known odorant ingredients of natural or synthetic origin. The range of the natural raw substances can embrace not only readily-volatile, but also moderately-volatile and slightly-volatile components and that of the synthetics can include representatives from practically all classes of fragrant substances, as will be evident from the following illustrative compilation: natural products, such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil, alcohols, such as farnesol, geraniol, linalool, nerol, phenylethyl alcohol, rhodinol, cinnamic alcohol, aldehydes, such as citral, Helional™, alpha-hexyl-cinnamaldehyde, hydroxycitronellal, Lilial™ (p-tert.butyl-alpha -methyldihydrocinnamaldehyde), methylnonylacetaldehyde, ketones, such as allylionone, alpha-ionone, beta - ionone, isoraldein (isomethyl- alpha -ionone), methylionone, esters, such as allyl phenoxyacetate, benzyl salicylate, cinnamyl propionate, citronellyl acetate, citronellyl ethoxolate, decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc., lactones, such as gamma-undecalactone, various components often used in perfumery, such as musk ketone, indole, p-menthane-8-thiol-3-one, and methyl-eugenol. Likewise, any conventional fragrant acetal or ketal known in the art can be added to the present composition as an optional component of the conventionally formulated perfume (c). Such conventional fragrant acetals and ketals include the well-known methyl and ethyl acetals and ketals, as well as acetals or ketals based on benzaldehyde, those comprising phenylethyl moieties, or more recently developed specialties such as those described in a United States Patent entitled "Acetals and Ketals of Oxo-Tetralins and Oxo-Indanes, see U.S. Pat. No. 5,084,440, issued January 28, 1992, assigned to Givaudan Corp. Of course, other recent synthetic specialties can be included in the perfume compositions for fully-formulated detergents. These include the enol ethers of alkyl-substituted oxo-tetralins and oxo-indanes as described in U.S. Pat. 5,332,725, July 26, 1994, assigned to Givaudan; or Schiff Bases as described in U.S. Pat. 5,264,615, December 9, 1991, assigned to Givaudan. It is preferred that the pro-fragrant material be added separately from the conventional fragrances to the detergent compositions of the invention.

Formulation with other Special-Purpose Fragrance Delivering Compounds

Detergents may further, optionally, if desired, contain other known compounds having the capability to enhance substantivity of a fragrance. Such compounds include, but are not limited to, the aluminium alkoxides such as isobutylaluminium diferanylate as disclosed in U.S. Pat. 4,055,634, issued October 25, 1977 and assigned to Hoffman-La Roch; or the known titanate and zirconate esters or oligoesters of fragrant materials such as those disclosed in U. S. Pat. 3,947,574, Jaggers et al, issued March 30, 1976 and U.S. 3,779,932, Jaggers, issued December 18, 1973. When using such organoaluminium, organotitanium or organozinc derivatives, they may be incorporated into the present formulations at their art-known levels.

Methods of Use

In its method aspect, the present invention can be described as:
A method of delivering residual fragrance to a washed surface, which comprises the steps of

(a) washing said surface in an aqueous solution of a detergent composition comprising

(i) a pro-fragrant compound selected from the group consisting of acetals, ketals, and mixtures thereof, said pro-fragrant compound having;

(1) a molecular weight of at least 350,
(2) a CLogP of at least 4, wherein CLogP is the logarithm to base 10 of the octanol/water partition coefficient of said pro-fragrant compound, and
(3) a half-life of less than 60 minutes, when measured at pH 0 by the Pro-Fragrant Hydrolysis Test; and

(ii) a detersive surfactant;

wherein said detergent composition has a pH of at least 7.1 when measured as a 1% solution in distilled-water at 20°C;
(b) subsequently exposing said surface to a reduction in pH.

EXAMPLES

EXAMPLE 1 (outside the scope of the invention)

Example of Process of Transacetalization

In a 500 ml single necked round bottom flask assembled with a short path distillation apparatus under nitrogen atmosphere, citral dimethyl acetal (41.0 g, 0.21 mol), geraniol (100 g, 0.65 mol, 3.2 eq.) and titanium isopropoxide (3.0 g, 5 mol %) are dissolved in 200 ml of toluene and brought to reflux. Toluene is distilled off as a means to azeotropically remove methanol from the reaction mixture. Six 150 ml portions of toluene are added to the reaction mixture and distilled off over the course of 10 hours until TLC shows the reaction is complete. The remaining toluene is removed under reduced pressure, and unreacted parent compounds are removed by bulb-to-bulb distillation at 65-85 °C, 0.4 mm Hg, yielding a yellow-brown oil. The product is then further purified by column chromatography on 230-400 mesh 60 A silica gel eluting with 2% ethyl acetate/ 1% triethylaminel petroleum ether yielding a yellow oil (59 g, 67% yield). t 1/2 at 0 pH is less than one minute. CLogP is 9.75.

EXAMPLE 2 (outside the scope of the present invention)

Example of Process of Acid Catalysis

In a 1 L single necked round bottom flask assembled with a Dean-Stark trap and condenser under a nitrogen atmosphere, decanal (50g. 32 mol.), geraniol (197.4g, 1.28 mol, 4 eq.).) and anhydrous citric acid (6.14g, 0.032 mol) are dissolved in 320 ml toluene and refluxed for 24 hours. Upon cooling, the reaction mixture is washed three times with saturated sodium carbonate followed by drying over magnesium sulfate. The solvent is removed under reduced pressure, and excess geraniol is removed under bulb-bulb distillation at 60-80° C, 0.1mm Hg, giving a clear yellow oil (132.1g, 92% yield). t $_{1/2}$ at 0 pH is 44 minutes. CLogP is 11.66.

EXAMPLE 3 (not within the scope of the present invention)

### Granular Laundry Composition delivering Geraniol from Digeranyl Citral Acetal

| | |
|---|---|
| Pro-fragrance of Example 1 | 1.0% |
| C11-C13 Dodecyl Benzene Sulfonate | 21.0% |
| C12-C13 Alkyl Ethoxylate EO 1-8 | 1.2% |
| Sodium Tripolyphosphate | 35.0% |
| Zeolite Na 4A | 14.0% |
| Sodium Silicate 2.0 ratio | 2.0% |
| Sodium Carbonate | 23.4% |
| Enzyme (Savinase™and/or Lipolase™ from Novo) | 1.4% |
| Carboxymethyl Cellulose | 0.3% |
| Anionic Soil Release Agent * | 0.3% |
| Brightener | 0.2% |
| Silicone Suds Suppressor ** | 0.2% |

| | |
|---|---|
| Perfume *** | 0.3% |
| Sodium Sulfate | 0.5% |
| Moisture | balance |

\* See U.S. 4,968,451

\*\* Commercial material available from Dow Corning Corp.

\*\*\*Perfume composition of the following formula:

| | |
|---|---|
| Benzyl salicylate | 20.0% |
| Ethylene brassylate | 20.0% |
| Galaxolide (50% soln. in | |
| benzyl benzoate) | 20.0% |
| Hexyl cinnamic aldehyde | 20.0% |
| Tetrahydro linalool | 20.0% |
| | 100% |

## Claims

1. A pro-fragrance compound selected from the group consisting of acetals, ketals, and mixtures thereof, wherein at least one of the parent aldehydes, ketones, or alcohols of said pro-fragrant acetal or ketal is a fragrance compound, said pro-fragrant compound having:

   (i) a molecular weight of at least about 350,
   (ii) a CLogP of at least about 4, wherein CLogP is the logarithm to base 10 of the Octanol/Water Partition Coefficient of said pro-fragrant compound, and
   (iii) a half-life of less than 60 minutes, when measured at pH 0 by the Pro-Fragrant Hydrolysis Test;

   provided that said parent aldehyde, ketone or alcohol of said acetal or ketal comprises at least one compound selected from the group consisting of;

   a) aldehydes, ketones and alcohols containing at least one aromatic moiety selected from the group consisting of $C_6H_4$ and $C_6H_3$ and wherein said parent aldehyde or ketone has a molecular weight of at least 125;
   b) monoalcohols selected from the group consisting of $C_{11}$-$C_{20}$ saturated, unsaturated, aromatic, aliphatic linear and branch chain alcohols and alkoxylates of said alcohols containing from 1 to about 30 alkoxy groups wherein said alkoxy is selected from the group consisting of ethoxy, propoxy and butoxy and mixtures thereof;
   c) polyhydroxy alcohols, and
   d) mixtures thereof;

   wherein said pro-fragrance compound is digeranyl p-t-bucinal acetal.

## Patentansprüche

1. Duftstoffvorläuferverbindung, gewählt aus der Gruppe, bestehend aus Acetalen, Ketalen und Mischungen hiervon, wobei mindestens einer der Ausgangsaldehyde, -ketone oder -alkohole des Duftstoffvorläuferacetals oder -ketals eine Duftstoffverbindung ist, wobei die Duftstoffvorläuferverbindung:

   (i) ein Molekulargewicht von mindestens etwa 350,

(ii) einen CLogP von mindestens etwa 4, worin CLogP der Logarithmus zur Basis 10 des Octanol/Wasser-Verteilungskoeffizienten der Duftstoffvorläuferverbindung ist, und

(iii) eine Halbwertszeit von weniger als 60 Minuten, gemessen bei pH 0 durch den Pro-Fragrant-Hydrolyse-Test, aufweist;

mit der Maßgabe, dass der Ausgangsaldehyd, -keton oder -alkohol des Acetals oder Ketals mindestens eine Verbindung umfasst, gewählt aus der Gruppe, bestehend aus

a) Aldehyden, Ketonen und Alkoholen, enthaltend mindestens eine aromatische Einheit, gewählt aus der Gruppe, bestehend aus $C_6H_4$ und $C_6H_3$, und wobei der Ausgangsaldehyd oder -keton ein Molekulargewicht von mindestens 125 besitzt;

b) Monoalkoholen, gewählt aus der Gruppe, bestehend aus gesättigten, ungesättigten, aromatischen, aliphatischen linearen oder verzweigtkettigen $C_{11}$-$C_{20}$-Alkoholen und Alkoxylaten dieser Alkohole, die 1 bis etwa 30 Alkoxygruppen enthalten, wobei das Alkoxy aus der Gruppe gewählt ist, bestehend aus Ethoxy, Propoxy und Butoxy und Mischungen hiervon;

c) Polyhydroxyalkoholen, und

d) Mischungen hiervon;

wobei die Dufstoffvorläuferverbindung Digeranyl-p-t-bucinalacetal ist.

## Revendications

1. Composé précurseur de parfum choisi dans le groupe constitué par les acétals, les cétals et des mélanges de ceux-ci, dans lequel au moins l'un des aldéhydes, cétones ou alcools parent(e)s dudit acétal ou cétal précurseur de parfum est un composé de parfum, ledit composé précurseur de parfum ayant :

(i) un poids moléculaire d'au moins environ 350,

(ii) un CLogP d'au moins environ 4, CLogP étant le logarithme de base 10 du coefficient de partage octanol/eau dudit composé précurseur de parfum, et

(iii) une demi-vie de moins de 60 minutes, lorsqu'elle est mesurée à pH 0 par l'essai d'hydrolyse de précurseur de parfum ;

étant entendu que ledit aldéhyde, ladite cétone ou ledit alcool parent(e) dudit acétal ou cétal comprend au moins un composé choisi dans le groupe constitué par ;

a) les aldéhydes, les cétones et les alcools contenant au moins un groupe aromatique choisi dans le groupe constitué par $C_6H_4$ et $C_6H_3$ et dans lequel ledit aldéhyde ou ladite cétone parent(e) a un poids moléculaire d'au moins 125 ;

b) les monoalcools choisis dans le groupe constitué par les alcools en $C_{11}$ à $C_{20}$ à chaîne linéaire et ramifiée, saturés, insaturés, aromatiques, aliphatiques et les alcoxylates desdits alcools contenant de 1 à environ 30 groupes alcoxy, dans lesquels ledit alcoxy est choisi dans le groupe constitué par éthoxy, propoxy et butoxy et des mélanges de ceux-ci ;

c) les alcools polyhydroxylés, et

d) des mélanges de ceux-ci ;

ledit composé précurseur de parfum étant le digéranyl p-t-bucinal acétal.